Europäisches Patentamt

(19) European Patent Office

Office européen des brevets

(11) Numéro de publication: **0 230 618**
**B1**

(12) # FASCICULE DE BREVET EUROPEEN

(45) Date de publication du fascicule du brevet:
**21.03.90**

(21) Numéro de dépôt: **86117626.1**

(22) Date de dépôt: **18.12.86**

(51) Int. Cl.⁴: **G01N 21/07, G01N 33/48**

---

(54) Procédé et dispositif pour délivrer une quantité prédéterminée de plasma à partir d'un échantillon de sang en vue d'analyses.

---

(30) Priorité: **20.12.85 FR 8518954**

(43) Date de publication de la demande:
**05.08.87 Bulletin 87/32**

(45) Mention de la délivrance du brevet:
**21.03.90 Bulletin 90/12**

(84) Etats contractants désignés:
**AT BE CH DE ES FR GB GR IT LI LU NL SE**

(56) Documents cités:
**EP-A- 0 062 907**
**EP-A- 0 084 983**
**EP-A- 0 160 282**
**FR-A- 2 524 874**

(73) Titulaire: **Guigan, Jean, 9, rue Jean Mermoz,**
**F-75008 Paris(FR)**

(72) Inventeur: **Guigan, Jean, 9, rue Jean Mermoz,**
**F-75008 Paris(FR)**

(74) Mandataire: **Weinmiller, Jürgen et al,**
**Lennéstrasse 9 Postfach 24, D-8133 Feldafing(DE)**

---

ACTORUM AG

## Description

La présente invention concerne un procédé et un dispositif pour délivrer une quantité prédéterminée de sérum ou plasma à partir d'un échantillon de sang en vue d'analyses.

Ce procédé et ce dispositif sont destinés notamment à venir compléter les procédés et dispositifs d'analyses décrits dans les brevets français n° 8416448 du 26 octobre 1984, n° 8419719 du 21 décembre 1984, n° 8504476 du 26 mars 1985, n° 8504477 du 26 mars 1985 correspondent à EP-A 183 627, EP-A 189 724, EP-A 197 865 et EP-A 197 866. Deux autres documents qui illustrent l'état de la technique sont: EP-A 160 282 et FR-A 2 524 874.

On sait que, pour les examens sanguins, on prélève du sang d'un malade dans un tube ayant un anticoagulant, on centrifuge le sang pour tasser les globules rouges au fond du tube et on effectue les analyses sur le plasma qui surmonte les globules. Les techniques d'analyses modernes ne nécessitant qu'une très faible quantité de plasma, le but de l'invention est de mettre en oeuvre un procédé et un dispositif permettant de transférer aux dispositifs d'analyses précités une très faible quantité de plasma, à partir d'une très faible quantité de sang, ce qui constitue un progrès très important pour le malade.

La présente invention a pour but un procédé pour délivrer une quantité prédéterminée de plasma à partir d'un échantillon de sang de quelques microlitres.
Un problème délicat dans le transfert de ces très faibles doses d'échantillons liquides résulte de l'utilisation de conduits capillaires reliant le réceptacle à différentes cellules. L'utilisation de la force centrifuge, qui se traduit par une augmentation de la densité apparente, permet de s'affranchir des forces capillaires de rétention.

La présente invention a pour objet un procédé pour délivrer une quantité prédéterminée de plasma à partir d'un échantillon de sang en vue d'analyses, caractérisé par le fait que l'on utilise :
- d'une part une barrette de transfert longitudinale en matière plastique transparente comportant un boîtier fermé par un couvercle, ledit foîtier comportant une première et une deuxième faces latérales extrêmes, une première et une deuxième faces longitudinales, et étant compartimenté de manière à présenter :
. le long de ladite première face longitudinale en partant de ladite première face extrême, une cuve réceptrice d'échantillon de sang, suivie d'un canal de sortie dans lequel aboutissent une cellule de stockage de plasma et une cellule de stockage de globules rouges,
. le long de ladite deuxième face lontitudinale en partant de ladite première face extrême, une cellule de mesure reliée par un tube capillaire d'une part à une cellule centrale d'évacuation de plasma ayant un orifice de sortie dans le fond dudit boîtier, et d'autre part à un canal de trop plein débouchant dans ladite cellule de stockage de plasma puis dans une cuve de trop plein, l'extrémité de la cellule de mesure opposée audit tube capillaire étant reliée par un conduit capillaire à des cellules de trop plein débouchant également dans ledit canal de trop plein,
- d'autre part un plateau tournant autour de son centre sur lequel ladite barrette peut être disposée radialement suivant deux positions A et B, la position A correspondant à ladite première face extrême plus proche du centre que ladite seconde face extrême et la positon B correspondant à une position symétrique de A par rapport au centre de ladite barrette, selon ce procédé :
- on introduit par un orifice prévu dans le couvercle d'une barrette un échantillon de sang de quelques microlitres qui tombe dans ladite cuve réceptrice, on dispose ladite barrette dans la position A sur ledit plateau tournant, la rotation dudit plateau faisant passer ledit échantillon de sang dans ladite cellule de stockage de plasma, dans ladite cellule de stockage de globules et dans ladite cuve de trop plein, la centrifugation entraînant l'apparition de plasma dans ladite cellule de stockage de plasma, et de globules rouges dans ladite cellule correspondante,
- on dispose ladite barrette dans la position B sur ledit plateau tournant, la rotation dudit plateau entraînant le remplissage de ladite cellule de mesure en plasma à partir de ladite cellule de stockage de plasma,
- on dispose ladite barrette dans la position A sur ledit plateau tournant, la rotation dudit plateau entraînant la vidange de ladite cellule de mesure et le passage d'une quantité calibrée de plasma dans ladite cellule d'évacuation puis vers l'extérieur dudit boîtier par ledit orifice.

Selon une variante de mise en oeuvre, on prévoit en outre dans ledit boîtier contre ladite deuxième face longitudinale et à la sortie desdites cellules de trop plein opposées à la sortie communiquant avec ladite cellule de mesure, un canal longitudinal aboutissant dans une cuve de stockage de plasma située au niveau de ladite seconde face extrême ; ainsi après la vidange d'une quantité calibrée de plasma,
- on dispose ladite barrete dans la position B pour remplir à nouveau ladite cellule de mesure à partir de ladite cuve de stockage,
- on dispose ladite barrette dans la position A pour vider ladite cellule de mesure dans ladite cellule d'évacuation.

La présente invention a également pour objet une barrette de transfert, caractérisée par le fait qu'elle comporte un boîtier fermé par un couvercle, tous deux en matière plastique transparente, ledit boîtier comportant une première et une deuxième faces latérales extrêmes, une première et une deuxième faces longitudinales, et étant compartimenté de manière à présenter :
. le long de ladite première face longitudinale en partant de ladite première face extrême, une cuve réceptrice d'échantillon de sang, suivie d'un canal de sortie dans lequel aboutissent une cellule de stockage de plasma et une cellule de stockage de globules rouges,
. le long de ladite deuxième face longitudinale en partant de ladite première face extrême, une cellule de mesure reliée par un tube capillaire d'une part à une cellule centrale d'évacuation de plasma ayant un orifice de sortie dans le fond dudit boîtier, et d'autre part à un canal de trop plein débouchant dans ladite cellule de stockage de plasma puis dans une cuve de trop plein, l'extrémité de la cellule de mesure opposée audit tube capillaire étant reliée par un conduit capillaire à des cellules de trop plein débouchant également dans ledit canal de trop plein. Selon une variante de réalisation, ledit boîtier comporte en outre contre ladite deuxième face longitudinale et à la sortie desdites cellules de trop plein opposées à la sortie communiquant avec ladite cellule de mesure, un canal longitudinal aboutissant dans une cuve de stockage de plasma située au niveau de ladite seconde face extrême.

D'autres caractéristiques et avantages de la présente invention apparaîtront au cours de la description suivante de modes de mise en oeuvre du procédé selon l'invention.

Dans le dessin annexé :

- Les figures 1A et 1B représentent schématiquement les deux positions A et B que peut adopter une barrette de transfert sur un plateau tournant conformément à l'invention.
- La figure 2 est une vue en perspective éclatée d'un exemple de barrette de transfert entrant dans la mise en oeuvre du procédé selon l'invention.
- Les figures 3 à 10 montrent en coupe, la barrette de transfert de la figure 2 en vue de dessus lors des différentes phases du transfert du plasma.
- La figure 11 est une vue en perspective éclatée d'un autre exemple de barrette de transfert permettant la mise en oeuvre du procédé selon l'invention.
- Les figures 12 et 23 montrent en coupe la barrette de transfert de la figure 11 en vue de dessus, lors des différentes phases de transfert du plasma.

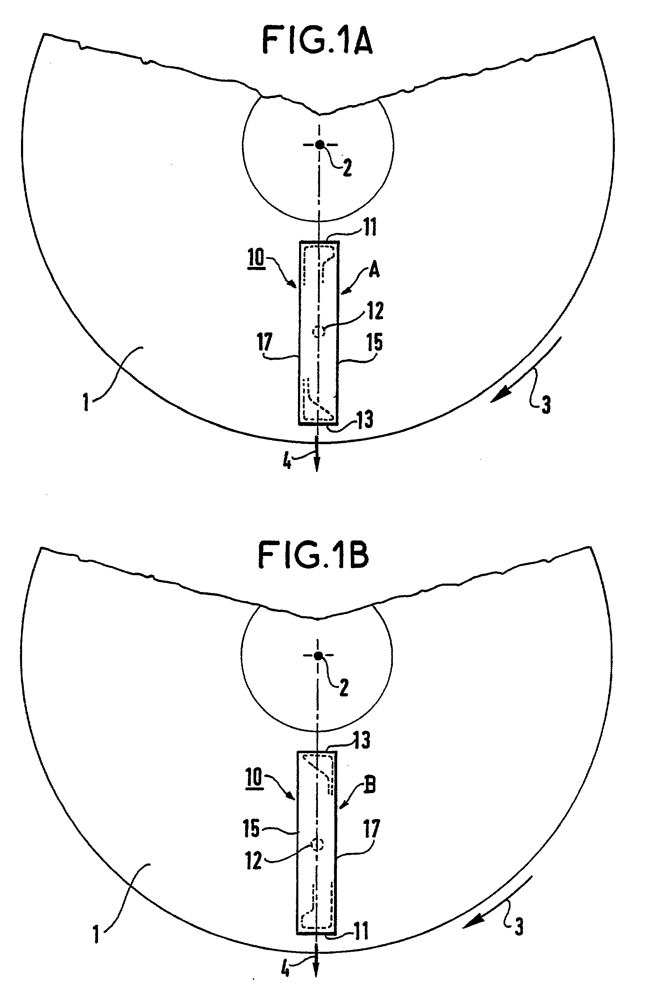

Sur les figures 1A et 1B la référence 1 désigne un plateau tournant susceptible d'être mis en rotation autour de son centre 2. La flèche 3 symbolise la rotation du plateau 1, et la flèche 4 indique la direction de la force centrifuge. Des barrettes de transfert 10 peuvent être disposées radialement sur ce plateau qui muni de moyens appropriés pour faire pivoter chaque barrette 10 d'une position radiale A, à une autre position radiale B symétrique de A par rapport au centre 12 de la barrette. On a référencé 11, 13, les faces latérales extrêmes de cette dernière, et 15, 17 ses faces longitudinales. A titre indicatif, la barrette 10 a une longueur de l'ordre de cinq centimètres, une largeur de l'ordre de un centimètre et l'on dispose six barrettes 10 sur le plateau 1 qui peut avoir vingt centimètres de diamètre environ.

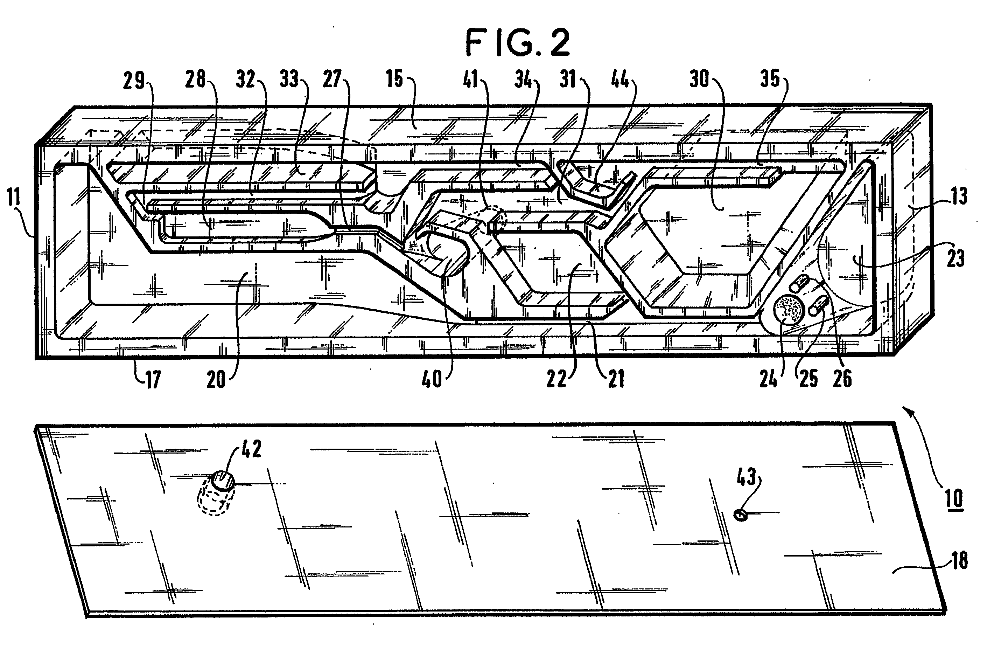

La barrette de transfert 10 que l'on voit en perspective dans la figure 2 comporte un boîtier fermé par un couvercle 18 ; l'ensemble est en matière plastique transparente. Le boîtier comporte un certain nombre de compartiments qui vont être explicités ci-dessous. De la face latérale extrême 11 à la face latérale extrême 13, on a le long de la face longitudinale 17 :
- une cuve réceptrice 20 pour l'échantillon de sang,
- un canal de sortie 21 issu de la cuve 20,
- une cellule de stockage de plasma 22 communiquant avec le canal 21,
- une cellule de stockage de globules rouges 23 communiquant avec l'extrémité du canal 21.

Une petite bille en matière plastique lestée 24, associée à deux pions de calage 25 et 26, est prévue au voisinage de l'orifice du canal 21 dans la cellule 23.

La cellule de stockage de plasma 22 est reliée d'une part par un tube capillaire 27 à une cellule de mesure 28 ayant un volume calibré, et d'autre part par un canal de trop plein 31 à une cuve de trop plein 30.

Vis à vis de la cellule de stockage de plasma 22, la cellule de mesure 28 est située du même côté que la cuve réceptrice 20, et la cuve de trop plein 30 est située du même côté que la cellule de stockage de globules 23.

La cellule de mesure 28 est, quant à elle, reliée d'une part par un conduit capillaire 29 à des cellules de trop plein 32, 33, eet d'autre part par le tube capillaire 27 à une cellule central d'évacuation de plasma 40 débouchant vers l'extérieur par un orifice de sortie 41 situé dans le fond de la barrette 10.

On a référencé 34 et 35 différents canaux de trop plein ; une chicane 44 est prévue entre les canaux 34 et 35.

Le couvercle 18 présente un orifice 42 pour l'introduction de l'échantillon de sang dans la cuve 20 et un évent 43.

Les cellules de trop-plein 32, 33, les canaux de trop plein 34, 35 et la cuve de top plein 30, sont disposés le long de la face longitudinale 15 de la barrette 10 quand on passe de la face latérale 11 à la face latérale 13.

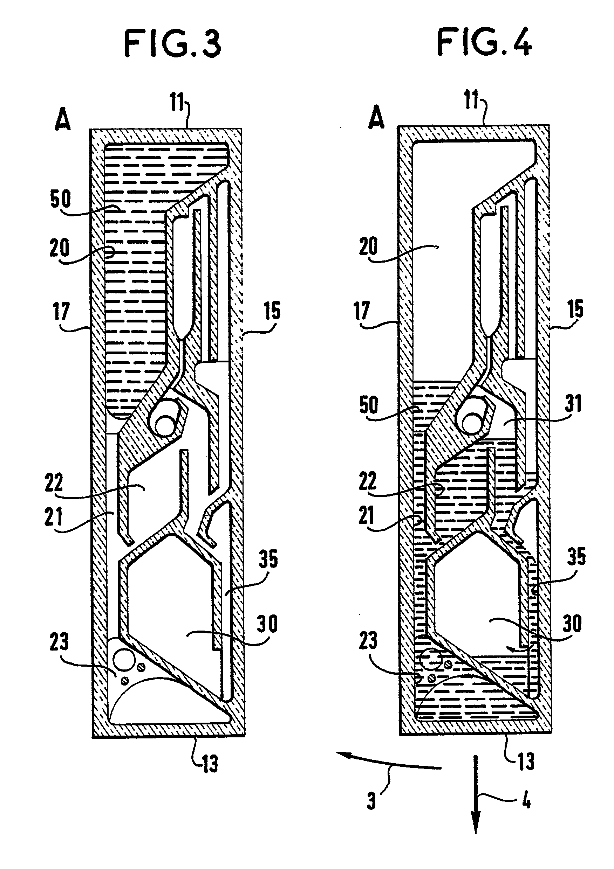

Les figures 3 à 6 montrent la barrette 10 dans la position 1 (figure 1A) pendant les différentes phases de la centrifugation.

Figure 3, la barrette 10 est à l'état de repos, et l'échantillon de sang 50 (quelques microlitres) se trouve dans la cuve réceptrice 20.

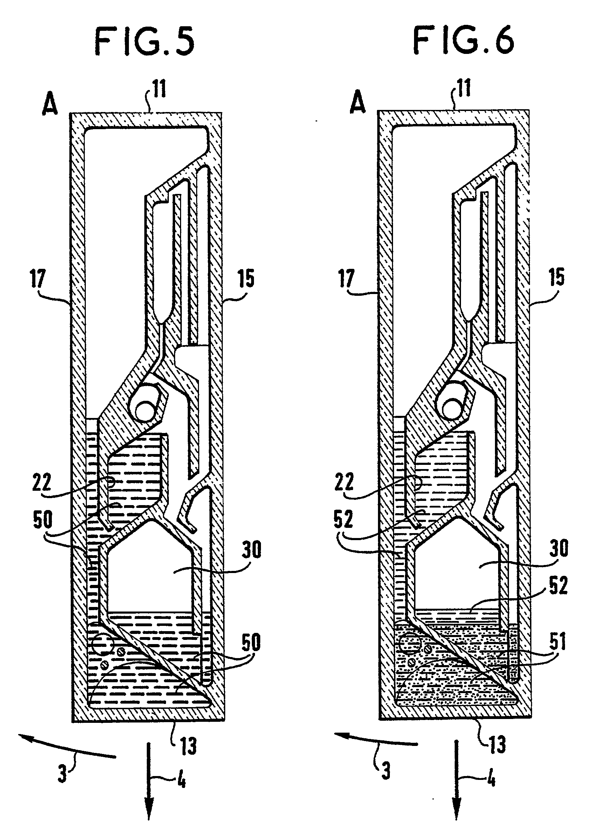

Figure 4, la centrifugation est en cours et, sous l'effet de la force centrifuge 4, le sang passe successivement de la cuve 20, au canal 21, remplit la cellule 23 ; puis il remplit complètement la cellule de stockage de plasma 22, les canaux de trop plein 31, 35 et partiellement la cuve de trop plein 30. On arrive alors à la situation illustrée par la figure 5.

Puis, la centrifugation se poursuit, et on observe la décantation et la séparation du sang en globules rouges 51 et en plasma 52 (figure 6).

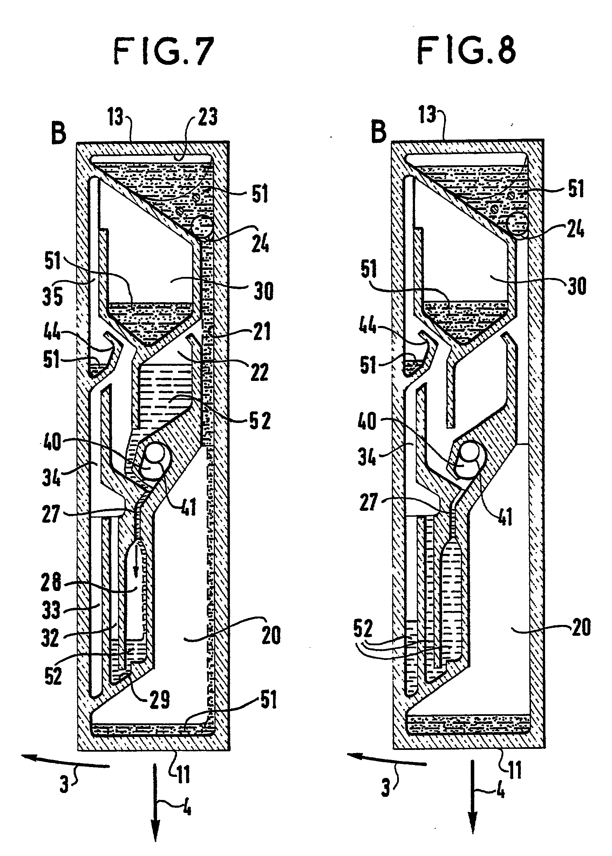

Lorsque la séparation est effectuée, la barrette est mise en position B (voir figure 1B). Comme on le voit sur les figures 7 et 8, la force centrifuge 4 a pour effet de vider la cellule de stockage 22 de son plasma qui remplit le tube capillaire 27, la cellule de mesure 28, puis les cellules de trop plein 32, 33. Le bille 24 vient obturer l'orifice faisant communiquer la cellule de stockage de globules rouges 23 avec le canal 21, mais elle laisse toutefois passer une petite quantité de globules 51 peu gênante, qui s'accumule contre la face extrême 11 de la cuve 20. La chicane 44, prévue sur le parcours du canal de trop plein 35, permet de piéger quelques globules rouges 51 qui pourraient sortir de la cuve de trop plein 30 et être entraînés vers les cellules 32, 33 contenant du plasma (figure 8).

Cette phase terminée, la barrette reprend la posi-

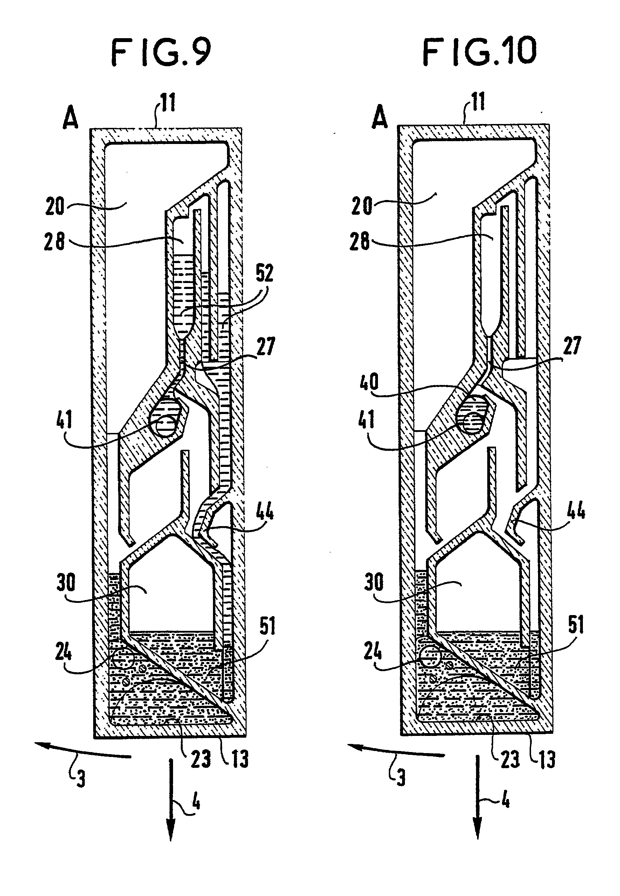

tion A (figure 1A). On parvient à la dernière phase du processus, (figures 9 et 10) c'est-à-dire au passage du volume calibré de plasma situé dans la cellule de mesure 28 dans la cellule d'évacuation 40, et à la sortie par l'orifice 41. Les globules rouges 51 sont maintenus dans la cellule 23 et contre une paroi de la cellule de trop plein 30.

La quantité calibrée de plasma évacuée par centrifugation peut aboutir dans un quelconque des dispositifs d'analyses connus et dont les exemples sont rappelés plus haut.

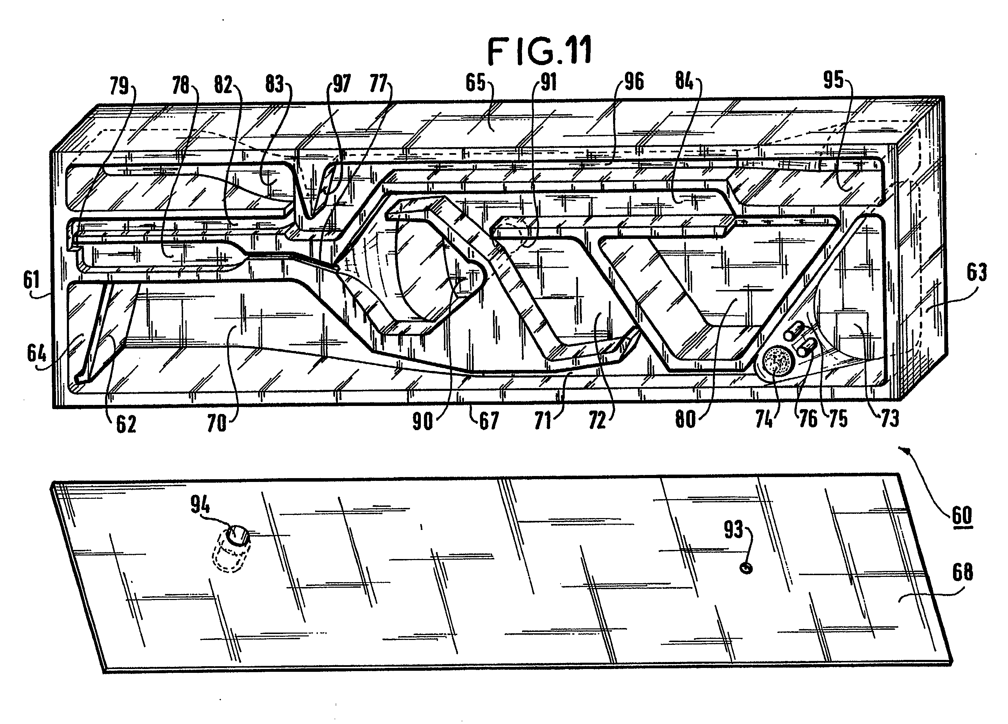

La figure 11 est une vue en perspective éclatée d'une variante de barrette de transfert 60 permettant de délivrer successivement deux doses calibrées de plasma.

Elle comporte un boîtier muni d'un couvercle 68 présentant un orifice 94 pour l'introduction de l'échantillon de sang et un évent 93.

On a référencé les faces latérales extrêmes 61 et 63 et les faces longitudinales 65 et 67.

Le faces 61 et 63 de la barrette 60 sont respectivement équivalentes aux faces 11 et 13 de la barrette 10, pour ce qui concerne les positions A et B prises sur le plateau tournant 1.

Le boîtier contient le long de sa face longitudinale 67, une cuve réceptrice de sang 70, un canal de sortie 71 dans lequel débouchent une cellule de stockage de plasma 72, puis une cellule de stockage de globules rouges 73.

La cuve 70 présente une paroi transversale supplémentaire 62 cloisonnant partiellement cette cuve et définissant un compartiment extrême 64.

Une petite bille en matière plastique lestée 74, associèe à deux pions de calage 75 et 76 est prévue au voisinage de l'orifice du canal 71 dans le cellule 73.

La cellule de stockage de plasma 72 est reliée d'une part par un tube capillaire 77 à une cellule de mesure 78 ayant un volume calibré, et d'autre part par un canal de trop plein 84 à une cuve de trop plein 80.

Vis à vis de la cellule de stockage de plasma 72, la cellule de mesure 78 est située du même côté que la cuve réceptrice 70, et la cuve de trop plein 80 est située du même côté que la cellule de stockage de globules 73.

La cellule de mesure 78 est reliée d'une part par un conduit capillaire 79 à des cellules de trop plein de plasma 82 et 83, et d'autre part par le tube capillaire 77 à une cellule centrale d'évacuation de plasma 90, débouchant vers l'extérieur par un orifice de sortie 91 situé dans le fond de la barrette 60.

Le long de la face longitudinale 65, sont disposés à partir de la face 61, une cellule de trop plein 83, un conduit 96, et une cuve de stockage de plasma 95 dont on verra le rôle plus loin. Une chicane 97 est prévue dans le conduit 96 au droit de l'entrée de la cellule de trop plein 83.

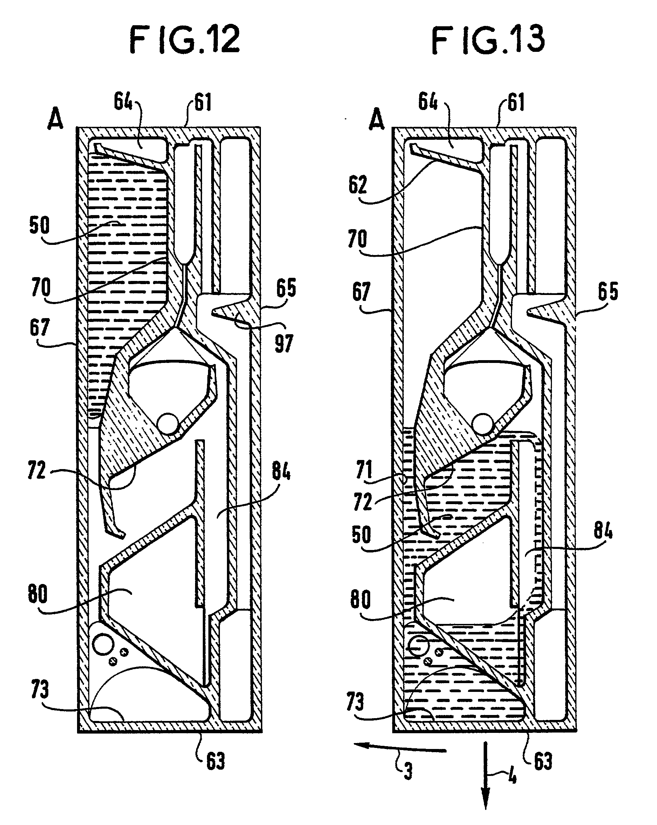

Les figures 12 à 15 montrent la barrette 60 dans la position A sur le plateau tournant (figure 1A).

Dans la figure 12, la barrette 60 est au repos et un échantillon de sang 50 se trouve dans la cuve 70.

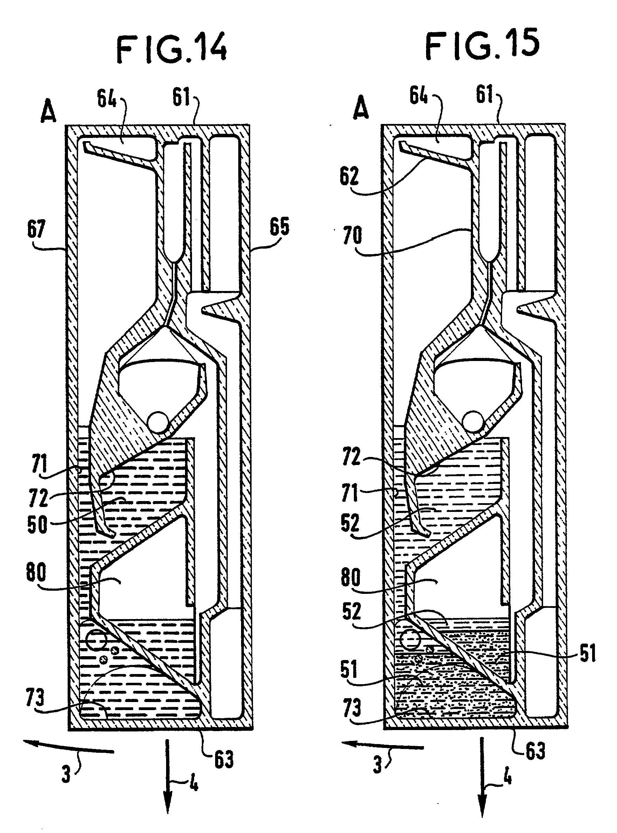

Dans la figure 13, la rotation 3 du plateau 1 a commencé et la force centrifuge 4 a pour effet de vider la cuve 70 par le canal 71 dans la cellule de stockage de plasma 72, la cellule 73, le canal de trop plein 84 et la cuve de trop plein 80. La figure 14 montre l'état de la barrette à ce moment. Par la poursuite de la rotation, (figure 15) on aboutit à une décantation et à l'accumulation de globules rouges 51 dans la cellule de stockage 73 et dans la cuve de trop plein 80. Du plasma 52 se trouve dans la cellule 72, dans le canal 71 et dans la cuve de trop plein 80.

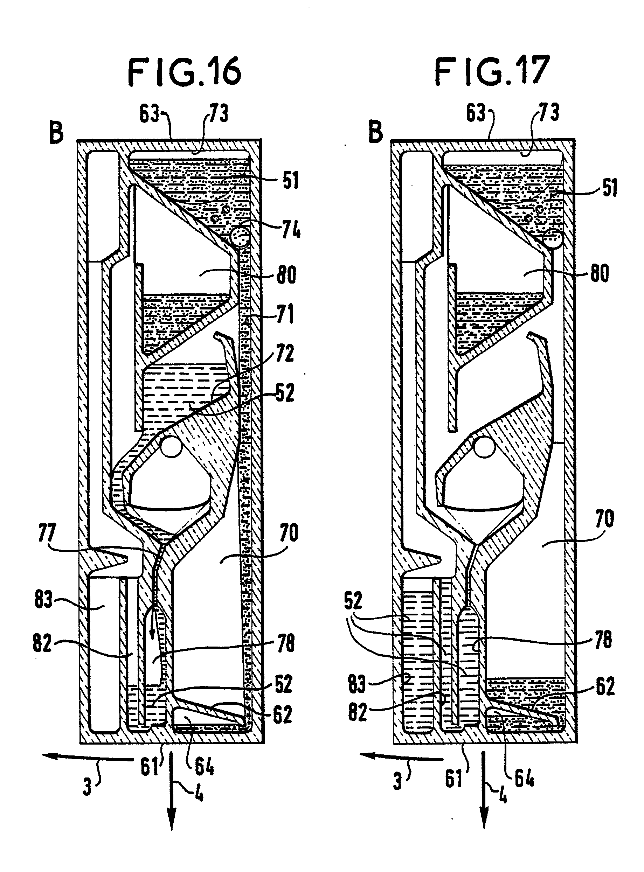

La barrette 60 est alors mise en position B (figure 1B). La force centrifuge 4 provoque alors la vidange de la cellule 72 par le tube capillaire 77 (figure 16).

Le plasma remplit tout d'abord la cellule de mesure 78, puis les cellules de trop plein 82 et 83. On aboutit à la situation illustrée dans la figure 17.

Dans le même temps quelques globules rouges présents dans la cellule 73 sont passés, malgré l'obturation du canal 71 par la bille 74, dans la cuve 70 et le compartiment 64.

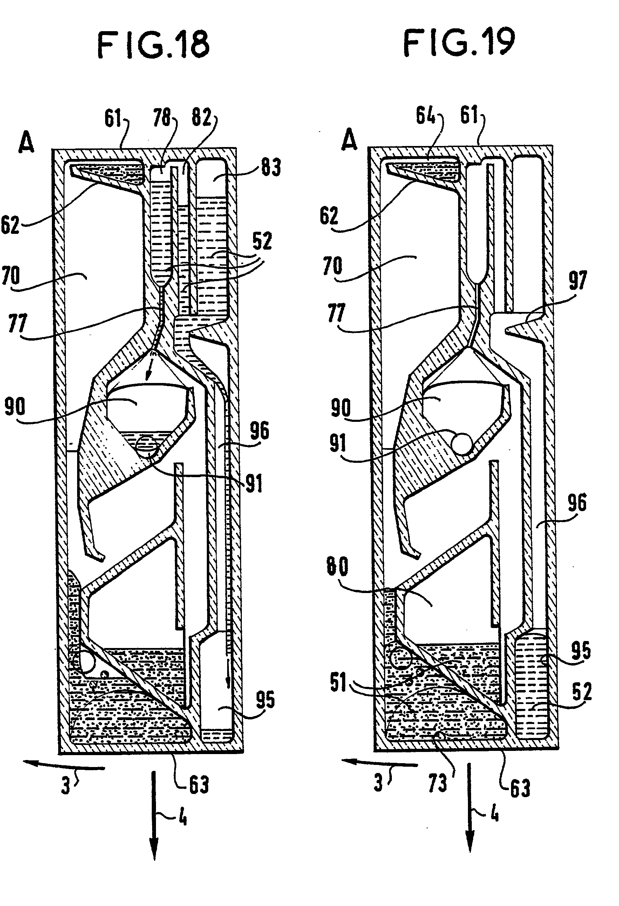

La barrette 60 est alors remise en position A (figure 1A). Il en résulte, grâce à l'effet de la force centrifuge 4, le passage d'une quantité calibrée de plasma 52 de la cellule calibrée 78 vers la cellule d'évacuation 90, par l'intermédiaire du tube capillaire 77 (figure 18). Cette quantité calibrée est évacuée par l'orifice de sortie 91. (figure 19). Simultanément l'éxcès de plasma présent dans les cellules de trop plein aboutit par le conduit 96 dans une cuve de stockage de plasma 95. Les globules rouges, quant à eux, sont stockés, dans la cellule 73, la cuve de trop plein 80 et le compariment 64.

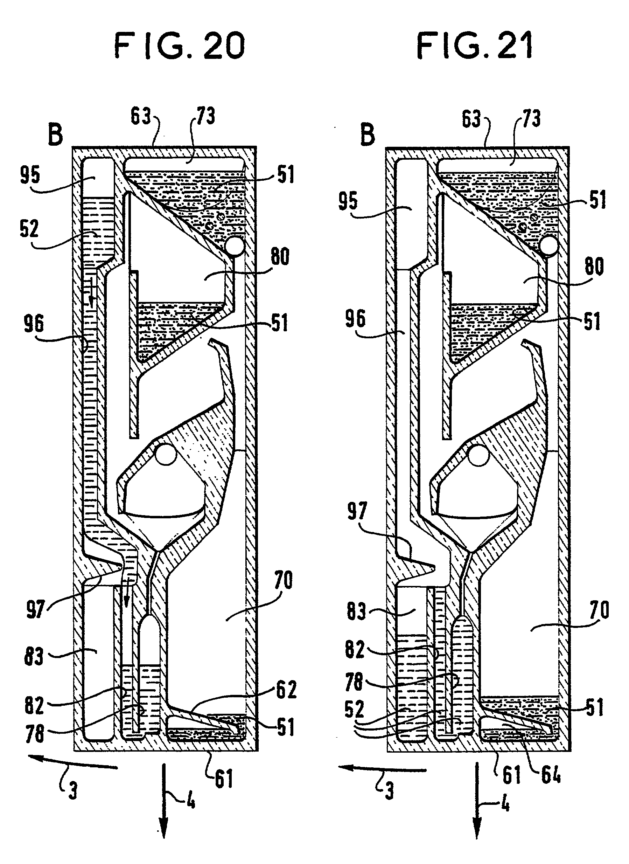

Pour obtenir une seconde dose calibrée de plasma, on remet la cuve 95 passe par le conduit 86 et remplit la cellule 82 et la cellule de mesure 78. La chicane 97 évite l'introduction préalable de plasma dans la cellule 83 (figure 20).

La figure 21 illustre la fin de cette phase.

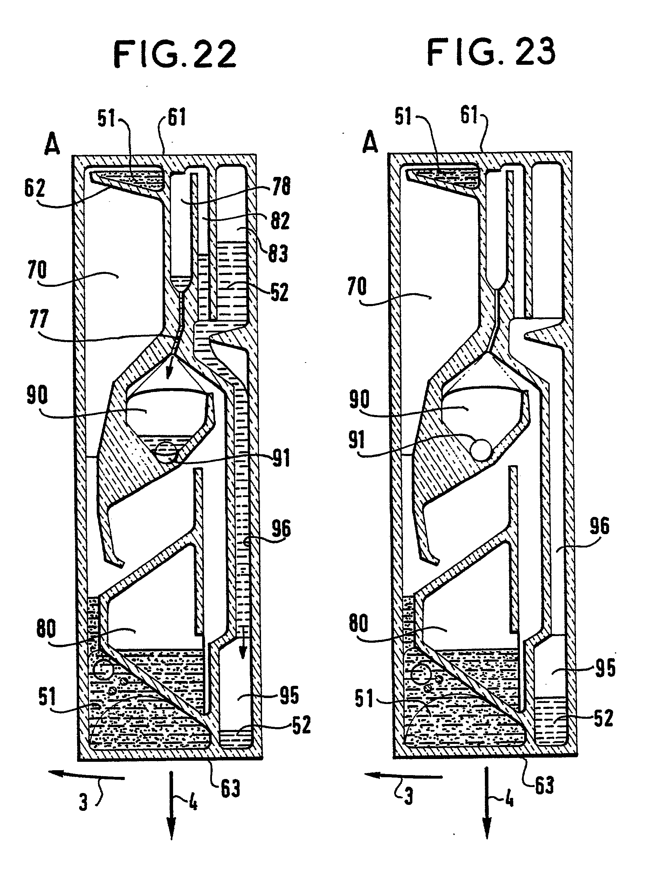

Enfin, on dispose à nouveau la barrette en position A, et on retrouve la situation illustrée dans les figures 18 et 19 (voir figures 22 et 23). Une dose calibrée de plasma 52 issue de la cellule de mesure 78 est évacuée par l'orifice 91 de la cellule 90.

S'il reste suffisamment de plasma dans la cuve 95 on peut encore renouveler le même opération.

Bien entendu, on pourra, sans sortir du cadre de l'invention remplace tout moyen par un moyen équivalent. Une barrette selon l'invention peut être réalisée aisément par moulage.

## Revendications

1. Procédé pour délivrer au moins une quantité prédéterminée de plasma à partir d'un échantillon de sang en vue d'analyses, caractérisé par le fait que l'on utilise :
- d'une part une barrette de transfert longitudinale en matière plastique transparente comportant un boîtier fermé par un couvercle, ledit boîtier comportant une première (11) et une deuxième (13) faces latérales extrêmes, une première (17) et une deuxième (15) faces longitudinales, et étant compartimenté de manière à présenter :
. le long de ladite première face longitudinale (17) en partant de ladite première face extrême (11), une cuve réceptrice (20) d'échantillon de sang, suivie d'un canal de sortie (21) dans lequel aboutissent une cellule de stockage de plasma (22) et une cellule de stockage de globules rouges (23),

. le long de ladite deuxième face longitudinale (15) en partant de ladite première face extrême, une cellule de mesure (28) reliée par un tube capillaire (27) d'une part à une cellule centrale d'évacuation de plasma (40) ayant un orifice de sortie (41) dans le fond dudit boîtier, et d'autre part à un canal de trop plein (31) débouchant dans ladite cellule de stockage de plasma (22) puis dans une cuve de trop plein (30), l'extrémité de la cellule de mesure (28) opposée audit tube capillaire (27) étant reliée par un conduit capillaire (29) à des cellules de trop plein (32, 33) débouchant également dans ledit canal de trop plein (31),

- d'autre part un plateau tournant autour de son centre sur lequel ladite barrette peut être disposée radialement suivant deux positions A et B, la position A correspondant à ladite première face extrême (11) plus proche du centre que ladite seconde face extrême (13) et la position B correspondant à une position symétrique de A par rapport au centre (12) de ladite barrette, selon ce procédé :

- on introduit par un orifice (42) prévu dans le couvercle d'une barrette (10) un échantillon de sang (50) de quelques microlitres qui tombe dans ladite cuve réceptrice,

- on dispose ladite barrette dans la position A sur ledit plateau tournant, la rotation dudit plateau faisant passer ledit échantillon de sang (50) dans ladite cellule de stockage de plasma (22), ladite cellule de stockage de globules (23) et dans ladite cuve de trop plein (30), la centrifugation entraînant l'apparition de plasma (52) dans ladite cellule de stockage de plasma (22) et de globules rouges (51) dans ladite cellule correspondante (23),

- on dispose ladite barrette dans la position B sur ledit plateau tournant, la rotation dudit plateau entraînant le remplissage de ladite cellule de mesure (28) en plasma à partir de ladite cellule de stockage de plasma (22),

- on dispose ladite barrette dans la position A sur ledit plateau tournant, la rotation dudit plateau entraînant la vidange de ladite cellule de mesure et le passage d'une quantité calibrée de plasma dans ladite cellule d'évacuation (40) puis vers l'extérieur dudit boîtier par ledit orifice (41).

2. Procédé selon la revendication 1, caractérisé par le fait que l'on prévoit en outre dans ledit boîtier contre ladite deuxième face longitudinale (65) et à la sortie desdites cellules de trop plein (82, 83) opposées à la sortie communiquant avec ladite cellule de mesure (78), un canal longitudinal (96) aboutissant dans une cuve de stockage de plasma (95) située au niveau de ladite seconde face extrême (63), et qu'ainsi après la vidange d'une quantité calibrée de plasma,

- on dispose ladite barrette dans la position B pour remplir à nouveau ladite cellule de mesure (78) à partir de ladite cuve de stockage (95),

- on dispose ladite barrette dans la position A pour vider ladite cellule de mesure (78) dans ladite cellule d'évacuation (90).

3. Barrette de transfert pour la mise en oeuvre du procédé selon la revendication 1, caractérisé par le fait qu'elle comporte un boîtier fermé par un couvercle, tous deux en matière plastique transparente, ledit boîtier comportant une première (11) et une deuxième (13) faces latérales extrêmes, une première (17) et une deuxième (15) faces longitudinales, et étant compartimenté de manière à présenter :

. le long de ladite première face longitudinale (17) en partant de ladite première face extrême (11), une cuve réceptrice (20) d'échantillon de sang, suivie d'un canal de sortie (21) dans lequel aboutissent une cellule de stockage de plasma (22) et une cellule de stockage de globules rouges (23),

. le long de ladite deuxième face longitudinale (15) en partant de ladite première face extrême, une cellule de mesure (28) reliée par un tube capillaire (27) d'une part à une cellule central d'évacuation de plasma (40) ayant un orifice de sortie (41) dans le fond dudit boîtier, et d'autre part à un canal de trop plein (31) débouchant dans ladite cellule de stockage de plasma (22) et dans une cuve de trop plein (30), l'extrémité de la cellule de mesure (28) opposée audit tube capillaire (27) étant reliée par un conduit capillaire (29) à des cellules de trop plein (32, 33) débouchant également dans ledit canal de trop plein (31).

4. Barrette de transfert selon la revendication 3, caractérisé par le fait que ledit boîtier comporte en outre contre ladite deuxième face longitudinale (65) et à la sortie desdites cellules de trop plein (82, 83) opposées à la sortie communiquant avec ladite cellule de mesure (78), un canal longitudinal (96) aboutissant dans une cuve de stockage de plasma (95) située au niveau de ladite seconde face extrême (63).

**Patentansprüche**

1. Verfahren zum Bereitstellen mindestens einer vorherbestimmten Menge an Plasma aus einer Blutprobe für die Analyse, gekennzeichnet durch die Verwendung

– einerseits eines länglichen Gefäßes aus durchsichtigem Kunststoff, das ein durch einen Deckel geschlossenes Gehäuse umfaßt, wobei das Gehäuse eine erste (11) und eine zweite (13) seitliche Endseite sowie eine erste (17) und eine zweite (15) Längsseite aufweist und derart unterteilt ist, daß es folgende Ausnehmungen aufweist:

. einen Blutprobenaufnahmetank (20), der sich entlang der ersten Längsseite (17) und beginnend an der ersten Endseite (11) erstreckt und an den sich ein Auslaßkanal (21) anschließt, in den eine Plasmaspeicherzelle (22) und eine Speicherzelle (23) für rote Blutkörperchen einmündet,

. eine Meßzelle (28), die sich entlang der zweiten Längsseite (15) und beginnend bei der ersten Endseite erstreckt und die über ein Kapillarrohr (27) einerseits an eine zentrale Plasmaentleerungszelle (40) mit einer Auslaßöffnung (41) im Boden des Gehäuses und andererseits an einen Überlaufkanal (31) angeschlossen ist, der in die Plasmaspeicherzelle (22) und dann in einen Überlauftank (30) einmündet, wobei das dem Kapillarrohr (27) gegenüberliegende Ende der Meßzelle (28) durch eine Kapillarleitung (29) mit Überlaufzellen (32, 33) verbunden ist, welche ebenfalls in den Überlaufkanal (31) einmünden,

– andererseits einer um ihre Mittelachse drehenden Scheibe, auf welcher das Gefäß radial entsprechend zwei Stellungen A und B angeordnet werden kann, wobei die Stellung A derjenigen entspricht, bei der sich die erste Endseite (11) näher bei der Mitte befindet als die zweite Endseite (13), während die Stellung B eine zur Stellung A symmetrischen Stellung in bezug auf die Mitte (12) des Gefäßes entspricht, wobei das Verfahren die folgenden Schritte aufweist:

– Einführen einer Blutprobe (50) von einigen Mikrolitern durch eine im Deckel des Gefäßes (10) vorgesehene Öffnung (42), die in den Auffangtank fällt,

– Anbringen des Gefäßes in Stellung A auf der Drehscheibe, wobei die Drehung der Scheibe die Blutprobe (50) in die Plasmaspeicherzelle (22), in die Speicherzelle (23) für die Blutkörperchen und in den Überlauftank (30) eintreten läßt, und das Zentrifugieren das Plasma (52) in die Plasmaspeicherzelle (22) und die roten Blutkörperchen (51) in die entsprechende Zelle eindringen läßt,

– Anordnen des Gefäßes in Stellung B auf der Drehscheibe, wobei die Drehung der·Scheibe das Füllen der Meßzelle (28) mit Plasma aus der Plasmaspeicherzelle (22) bewirkt,

– Anordnen des Gefäßes in Stellung A auf der Drehscheibe, wobei die Drehung der Scheibe die Entleerung der Meßzelle und das Übertreten einer abgemessenen Menge an Plasma in die Entleerungszelle (40) und durch die genannte Öffnung (41) weiter aus dem Gehäuse hinaus bewirkt.

2. Verfahren nach Anspruch 1, dadurch gekennzeichnet, daß im Gehäuse weiter entlang der zweiten Längsseite (65) und am Ausgang der Überlaufzellen (82, 83), die zu dem mit der Meßzelle (78) in Verbindung stehenden Ausgang entgegengesetzt liegen, ein Längskanal (96) vorgesehen ist, der in einen an der zweiten Endseite (63) liegenden Plasmaspeichertank (95) einmündet, so daß nach dem Ablassen einer abgemessenen Menge an Plasma

– das Gefäß zum erneuten Füllen der Meßzelle (78) aus dem Speichertank (95) in die Stellung B gebracht wird, und

– das Gefäß zum Leeren der Meßzelle (78) in die Entleerungszelle (90) in die Stellung A gebracht wird.

3. Transfer-Gefäß zur Durchführung des Verfahrens nach Anspruch 1, dadurch gekennzeichnet, daß es einen durch einen Deckel geschlossenen Behälter aufweist, die beide aus durchsichtigem Kunststoff bestehen, wobei das Gehäuse eine erste (11) und eine zweite (13) seitliche Endseite sowie eine erste (17) und eine zweite Längsseite (15) besitzt und derart unterteilt ist, daß es folgende Ausnehmungen aufweist:

. einen Blutprobenaufnahmetank (20), der sich entlang der ersten Längsseite (17) beginnend an der ersten Endseite (11) erstreckt und an den sich ein Auslaßkanal (21) anschließt, in den eine Plasmaspeicherzelle (22) und eine Speicherzelle (23) für rote Blutkörperchen einmünden,

. eine Meßzelle (28), die sich entlang der zweiten Endseite (15) beginnend bei der ersten Endseite erstreckt und die über ein Kapillarrohr (27) einerseits an eine Plasmaentleerungszelle (40) mit einer Auslaßöffnung (41) im Boden des Gehäuses, und andererseits an einen Überlaufkanal (31) angeschlossen ist, der in die Plasmaspeicherzelle (22) und dann in einen Überlauftank (30) einmündet, wobei das dem Kapillarrohr (27) gegenüberliegende Ende der Meßzelle (28) durch eine Kapillarleitung (29) mit Überlaufzellen (32, 33) verbunden ist, welche ebenfalls in den Überlaufkanal (31) einmünden.

4. Transfer-Gefäß nach Anspruch 3, dadurch gekennzeichnet, daß das Gehäuse weiter entlang der zweiten Längsseite (65) und am Ausgang der Überlaufzellen (82, 83), welche dem mit der Meßzelle (78) verbundenen Ausgang gegenüberliegen, einen Längskanal (96) aufweist, der in einen Plasmaspeichertank (95) an der zweiten Endseite (63) einmündet.

## Claims

1. A method of delivering a predetermined quantity of plasma from a blood sample for analysis purposes, the method being characterized in that the following items are used:

– on the one hand a longitudinal transfer bar made of transparent plastic material and comprising a housing enclosed by a lid, said housing comprising first (11) and second lateral end faces (13), first (17) and second longitudinal faces (15), and being compartmented so as to present:

. a receptacle (20) for receiving a blood sample disposed along said first longitudinal face (17) and starting from said first end face (11) and followed by an outlet channel (21) into which a plasma storage cell (23) opens out; and

. a measurement cell (28) disposed along said second longitudinal side face (15) and starting from said first end face and connected by a capillary tube (27) on the one hand to a plasma removal cell (40) having an outlet orifice (41) through the bottom of said housing, and on the other hand to an overflow channel (31) opening out into said plasma storage cell (22) and then into an overflow tank (30), the end of the measurement cell (28) opposite to said capillary tube (27) being connected by a capillary duct (29) to overflow cells (32, 33) which also open out into said overflow channel (31);

– and on the other hand a disk rotatable about its center and on which said bar may be disposed radially in two positions A and B, the position A corresponding to said end face (11) being closer to the center of the disk than said second end face (13), and the position B being symmetrical to the position A about the middle of said bar; said method comprising the steps of:

– inserting a sample of a few microliters of blood (50) through an orifice provided in the lid of a bar (10) so that said blood drops into said receptacle;

– disposing said bar in said A position on said disk and rotating said disk to cause said sample of blood (50) to pass into the said plasma storage cell (22), said corpuscle storage cell (23) and said overflow tank (30), the centrifuging causing plasma to appear in said plasma storage cell (22) and red corpuscles to appear in said corresponding corpuscle storage cell (23);
– placing said bar in the B position on said rotating disk, the rotation of said disk causing said measurement cell (28) to be filled with plasma from said plasma storage cell (22), and
– placing said bar in said A position on said rotating disk, the rotation of said disk causing said measurement cell to be emptied and causing a calibrated quantity of plasma to pass into said removal cell (40) and thence out from said housing via said outlet orifice (41).

2. A method according to claim 1, characterized in that said housing further includes a longitudinal channel (96) extending along said second longitudinal face (65) and disposed at the outlet from said overflow cells (82, 83) opposite to the outlet communicating with the measurement cell (78), this channel opening out into a plasma storage tank (95) situated level with said second end face (63), such that after delivering a calibrated quantity of plasma,
– said bar is placed in the B position in order to refill said measurement cell (78) from said storage tank (95), and
– said bar is again placed in said A position in order to empty said measurement cell (78) into said removal cell (90).

3. A transfer bar for implementing the method according to claim 1, characterized in that it comprises a housing closed by a lid, both of which are made of transparent plastic material, said housing comprising first (11) and second lateral end faces (13), first (17) and second side faces (15), and being compartmented in such a manner as to present:
. a receptacle (20) for receiving a blood sample disposed along said first longitudinal face (17) and starting from said first end face (11), followed by an outlet channel (21) into which a plasma storage cell (22) and a red corpuscle storage cell (23) opens out; and
. a measurement cell (28) disposed along said second longitudinal face (15) and starting from said first end face and connected by a capillary tube (27) on the one hand to a central plasma removal cell (40) having an outlet orifice (41) through the bottom of said housing, and on the other hand to an overflow channel (31) opening out into said plasma storage cell (22) and into an overflow tank (30), the end of the measurement cell (28) opposite to said capillary tube (27) being connected by a capillary duct (29) to overflow cells (32, 33) which also open out into said overflow channel (31).

4. A transfer bar according to claim 3, characterized in that said housing further includes a longitudinal channel (96) extending along said second longitudinal face (65) and disposed at the outlet from said overflow cells (82, 83) opposite to the outlet communicating with the measurement cells (78), this channel opening out into a plasma storage tank (95) situated level with said second end face (63).

# FIG.1A

# FIG.1B

# FIG. 2

EP 0 230 618 B1

# FIG.3

# FIG.4

EP 0 230 618 B1

# FIG.5

A

11
17
15
22
50
30
50
13
3
4

# FIG.6

A

11
17
15
22
52
30
52
51
13
3
4

# FIG.7

# FIG.8

# FIG.9

# FIG.10

FIG.11

# FIG.12

# FIG.13

# FIG.14

# FIG.15

# FIG.16

# FIG.17

# FIG.18

# FIG.19

# FIG. 20

# FIG. 21

FIG.22    FIG.23